# EUROPEAN PATENT APPLICATION

(11) **EP 4 350 905 A1**
(43) Date of publication of application: **10.04.2024**
(21) Application number: 22811008.6
(22) Date of filing: 29.03.2022
(51) Int. Cl.: H01S 3/08, H01S 3/082, H01S 3/0941, H01S 3/113, H01S 5/183

(54) **LASER ELEMENT AND ELECTRONIC DEVICE**

(30) Priority: 26.05.2021 JP 2021088669
(71) Applicant: Sony Group Corporation, Tokyo 108-0075 (JP)
(72) Inventor: KAMATA, Masanao, Tokyo 108-0075 (JP); YONEZAWA, Gen, Tokyo 108-0075 (JP)
(74) Representative: D Young & Co LLP
(86) International application number: PCT/JP2022/015467
(87) International publication number: WO 2022/249733

(57) **Abstract**

A temperature rise in an optical resonator can be suppressed, or damage to the optical resonator due to laser light condensing can be avoided.

A laser element includes a semiconductor layer including a first reflection layer with respect to a first wavelength and an active layer that performs surface emission at the first wavelength, a laser medium arranged on a rear side of an optical axis of the semiconductor layer and including a second reflection layer with respect to a second wavelength on a first surface facing the semiconductor layer and a third reflection layer with respect to the first wavelength on a second surface on a side opposite to the first surface, a fourth reflection layer with respect to the second wavelength arranged on the second surface or arranged on a rear side of the optical axis with respect to the second surface, a first resonator that causes light having the first wavelength to resonate between the first reflection layer and the third reflection layer, a second resonator that causes light having the second wavelength to resonate between the second reflection layer and the fourth reflection layer, and an optical element arranged between the second reflection layer and the fourth reflection layer, the optical element that increases a beam diameter of the light having the second wavelength, in which the optical axis of the semiconductor layer, an optical axis of the laser medium, and an optical axis of the optical element are coaxially arranged.

## Description

### TECHNICAL FIELD

The present disclosure relates to a laser element and an electronic device.

### BACKGROUND ART

Peak power of a laser is defined as pulse energy ÷ pulse width, and in order to obtain higher peak power, it is important to obtain a shorter pulse width. A Q-switched solid-state laser that outputs a laser pulse has a feature that a length of its own resonator is proportional to the obtained pulse width, and a minimum resonator length is determined by a length of the used solid-state laser medium. Since the length of the solid-state laser medium installed in the resonator as a gain medium is determined by an absorption amount of excitation light, if the length of the solid-state laser medium is simply shortened, the excitation light cannot be sufficiently absorbed, and excitation efficiency is significantly deteriorated.

Therefore, in the conventional method of externally exciting a Q-switched solid-state laser medium with a semiconductor laser, it is not preferable to shorten the length of the solid-state laser medium than an excitation light absorption length, and a shorter pulse cannot be obtained. That is, when the solid-state laser medium length is shortened in order to obtain a short pulse width, the absorption amount of the excitation light decreases, and the excitation efficiency is deteriorated. In contrast, there has been a trade-off that increasing the solid-state laser medium length in order to increase the absorption amount of the excitation light increases the resonator length and increases the pulse width.

In contrast, from the viewpoint of manufacturing and light source output stability, the conventional Q-switched solid-state laser needs to perform assembly adjustment by positioning a plurality of optical elements with high accuracy, so that this is poor in mass productivity and has difficulty in cost reduction, and there is a problem in stability of light source output due to positional displacement of each optical element.

Conventionally, for example, there is known a method of generating a short-pulse laser by externally exciting a Q-switched solid-state laser using a semiconductor laser (refer to Patent Documents 1 and 2). Since the obtained pulse width is proportional to the resonator length of the Q-switched solid-state laser, it is desirable to shorten the resonator length to obtain a shorter pulse width in order to obtain higher laser peak power.

However, in the conventional method, a thickness of the solid-state laser medium that can be used is limited by the absorption length determined by a wavelength of the semiconductor laser for excitation and an absorption coefficient of the solid-state laser medium at the wavelength. For example, in a case of Nd:YAG (10 at%) that is generally most often used in a Q-switched solid-state laser, the absorption length for excitation light having a wavelength of 808 nm is about 10 mm; if the length of the solid-state laser medium is made shorter than this length, the remaining excitation light that has not been absorbed returns to the semiconductor laser side to make an operation unstable or cause heat generation. In a disk laser, a method of folding back the excitation light several times has also been proposed, but this requires a complicated excitation optical system, and there is a problem in achieving a compact size and a low cost.

Furthermore, conventionally, in order to make a laser light source compact, for example, as disclosed in Patent Document 3, a method of integrally stacking a surface emitting laser (VCSEL) for excitation and a solid-state laser medium has been proposed. However, there is only a description of "integrally "stacking, and there is no specific description of whether light transmission surfaces are joined to each other, which joining process is used, and how to solve the problem caused by this.

Moreover, conventionally, in order to compensate for reduction in mode size due to a thermal lens effect accompanying the temperature rise of the solid-state laser medium, for example, as disclosed in Patent Document 4, a structure adopting a convex surface that spreads light reflected by an output coupling mirror in an optical resonator has been proposed.

### CITATION LIST

### PATENT DOCUMENT

PATENT DOCUMENT 1 Japanese Patent Application Laid-Open No. 2013-219232
PATENT DOCUMENT 2 Japanese Patent Application Laid-Open No. 2019-176119
PATENT DOCUMENT 3 Japanese Patent Application Laid-Open No. 2007-173393
PATENT DOCUMENT 4 International Publication No. WO 2019/049694

### SUMMARY OF THE INVENTION

### PROBLEMS TO BE SOLVED BY THE INVENTION

However, in a case of Patent Document 4, the excitation light is separated and it has been necessary to guide the excitation light to an external resonator using a collimator lens and a condenser lens. Therefore, highly accurate positioning adjustment of each optical component including the output coupling mirror is required each time.

Therefore, the present disclosure provides a laser element capable of suppressing a temperature rise in an optical resonator or capable of avoiding damage to the optical resonator due to laser light condensing.

### SOLUTIONS TO PROBLEMS

In order to solve the above-described problem, according to the present disclosure, provided is a laser element including:
a laminated semiconductor layer including a first reflection layer with respect to a first wavelength and an active layer that performs surface emission at the first wavelength;
a laser medium arranged on a rear side of an optical axis of the laminated semiconductor layer and including a second reflection layer with respect to a second wavelength on a first surface facing the laminated semiconductor layer and a third reflection layer with respect to the first wavelength on a second surface on a side opposite to the first surface;
a fourth reflection layer with respect to the second wavelength arranged on the second surface or arranged on a rear side of the optical axis with respect to the second surface;
a first resonator that causes light having the first wavelength to resonate between the first reflection layer and the third reflection layer;
a second resonator that causes light having the second wavelength to resonate between the second reflection layer and the fourth reflection layer; and
an optical element arranged between the second reflection layer and the fourth reflection layer, the optical element that increases a beam diameter of the light having the second wavelength, in which
the optical axis of the laminated semiconductor layer, an optical axis of the laser medium, and an optical axis of the optical element are coaxially arranged.

An end face on a side of the laser medium of the laminated semiconductor layer may be joined to an end face on a side of the laminated semiconductor layer of the laser medium.

The optical element may reflect or refract at least a part of the light having the second wavelength so that the light having the second wavelength is not condensed.

The optical element may include a dielectric multilayer film.

The optical element may include a convex mirror that reflects at least a part of incident light, or a light refracting member that refracts at least a part of the incident light so that the incident light is not condensed.

In a case where the optical element includes the fourth reflection layer, the fourth reflection layer may be the convex mirror, and in a case where the optical element is provided on a side closer to the second reflection layer than the fourth reflection layer, the optical element may include the light refracting member.

The optical element may include a transparent member that flattens a side of a surface opposite to a reflection surface of the convex mirror or a refraction surface of the light refracting member and transmits the light having the second wavelength.

A light control member that is joined to the transparent member arranged on the side of the surface opposite to the refraction surface of the light refracting member and controls a refraction or polarization direction of the light having the second wavelength transmitted through the optical element may be included.

The optical element may have a fine periodic structure that reflects or refracts at least a part of incident light in such a manner that the incident light is not condensed, and
the fine periodic structure may include irregularities periodically arranged in a surface direction.

The fine periodic structure may be a Fresnel lens, a meta lens, or a photonic crystal lens.

The optical element may include a flat surface having a refractive index distribution in a surface direction that reflects or refracts at least a part of incident light in such a manner that the incident light is not condensed.

A saturable absorber including the fourth reflection layer on a third surface on a side opposite to the laser medium may be included, in which
the optical axis of the laminated semiconductor layer, the optical axis of the laser medium, the optical axis of the saturable absorber, and the optical axis of the optical element may be coaxially arranged.

The laminated semiconductor layer, the laser medium, and the saturable absorber may be integrally joined.

A first transparent medium arranged between the laminated semiconductor layer and the laser medium, the first transparent medium that transmits the light having the first wavelength may be included.

A second transparent medium arranged between the laser medium and the saturable absorber, the second transparent medium that transmits the light having the second wavelength may be included.

The optical element may be provided on at least one of the laser medium or the saturable absorber.

The optical element may be provided on a surface or inside of the laser medium and the saturable absorber.

The optical element may be provided along the third surface of the saturable absorber.

The optical element may be arranged between the laser medium and the saturable absorber.

The fourth reflection layer may be an output coupling mirror in the second resonator.

The laminated semiconductor layer may include a fifth reflection layer with respect to the first wavelength, the fifth reflection layer arranged on a side closer to the laser medium than the first reflection layer, and
the fifth reflection layer may transmit a part of the light having the first wavelength.

According to another aspect of the present disclosure, provided is an electronic device including:
a laser element; and
a control unit that performs control to emit light from the laser element, in which
the laser element includes:
   a laminated semiconductor layer including a first reflection layer with respect to a first wavelength and an active layer that performs surface emission at the first wavelength;
   a laser medium arranged on a rear side of an optical axis of the laminated semiconductor layer and including a second reflection layer with respect to a second wavelength on a first surface facing the laminated semiconductor layer and a third reflection layer with respect to the first wavelength on a second surface on a side opposite to the first surface;
   a fourth reflection layer with respect to the second wavelength arranged on the second surface or arranged on a rear side of the optical axis with respect to the second surface;
   a first resonator that causes light having the first wavelength to resonate between the first reflection layer and the third reflection layer;
   a second resonator that causes light having the second wavelength to resonate between the second reflection layer and the fourth reflection layer; and
   an optical element arranged between the second reflection layer and the fourth reflection layer, the optical element that increases a beam diameter of the light having the second wavelength, and
   the optical axis of the laminated semiconductor layer, an optical axis of the laser medium, and an optical axis of the optical element are coaxially arranged.

### BRIEF DESCRIPTION OF DRAWINGS

Fig. 1 is a diagram illustrating a basic configuration of a laser element according to the present disclosure.
Fig. 2 is a schematic cross-sectional view illustrating a first mode of the optical element.
Fig. 3 is a schematic cross-sectional view illustrating a second mode of the optical element.
Fig. 4A is a cross-sectional view illustrating an example of a third mode of the optical element.
Fig. 4B is a cross-sectional view illustrating another example of the third mode of the optical element.
Fig. 4C is a cross-sectional view illustrating another example of the third mode of the optical element.
Fig. 5A is a schematic cross-sectional view illustrating a fourth mode of the optical element.
Fig. 5B is a schematic cross-sectional view illustrating the fourth mode of the optical element.
Fig. 6 is a schematic cross-sectional view illustrating a fifth mode of the optical element.
Fig. 7 is a schematic cross-sectional view of a laser array in which the laser elements in Fig. 3 are arranged in an array.
Fig. 8 is a schematic cross-sectional view of a laser array in which the laser elements in Fig. 5 are arranged in an array.
Fig. 9 is a schematic cross-sectional view of a laser array in which the laser elements in Fig. 6 are arranged in an array.
Fig. 10 is a diagram schematically illustrating a manufacturing process of the laser element of the present disclosure.
Fig. 11 is a diagram illustrating an example in which a first transparent medium and a second transparent medium are arranged between an excitation light source and a solid laser medium.
Fig. 12 is a diagram illustrating a basic configuration of the laser element not including a saturable absorber.
Fig. 13 is a diagram illustrating the laser element obtained by providing the above-described optical element in the laser element in Fig. 12.
Fig. 14 is a diagram illustrating an example of a schematic configuration of an endoscope system.
Fig. 15 is a block diagram illustrating an example of a functional configuration of a camera and a CCU illustrated in Fig. 14.
Fig. 16 is a diagram illustrating an example of a schematic configuration of a microscopic surgery system.

### MODE FOR CARRYING OUT THE INVENTION

Hereinafter, an embodiment of a laser element is described with reference to the drawings. Although principal components of the laser element are mainly described hereinafter, the laser element might include components and have functions that are not illustrated or described. The following description does not exclude components and functions that are not illustrated or described.

### (Technical Features of Laser Element according to Present Disclosure)

First, before describing an internal configuration and an operation of a laser element according to the present disclosure, technical features of the laser element according to the present disclosure are described.

The laser element according to the present disclosure has a configuration in which a structure using a part of a surface emitting laser as an excitation light source and a solid-state laser medium for Q-switching are integrally joined. Note that, as described later, the laser element according to the present disclosure may include the laser element not having a Q-switching function, but the laser element having the Q-switching function is first described.

In the laser element according to the present disclosure, the solid-state laser medium for Q-switching is shared by two resonators. These two resonators include a first resonator that resonates at a first wavelength and a second resonator (also referred to as a Q-switched solid-state laser resonator) that resonates at a second wavelength.

Since the two resonators share the solid-state laser medium, high-intensity excitation of the solid-state laser medium can be performed in the first resonator even when the solid-state laser medium is shortened, and a laser pulse having a shorter pulse width can be generated.

Furthermore, since the laser element according to the present disclosure is an integrated laminated structure that can be fabricated by using a semiconductor process technology, this is excellent in mass productivity and also excellent in laser output stability.

Here, the excitation light source is a mode of a vertical cavity surface emitting laser (VCSEL). This is different from the VCSEL in that at least one of mirrors forming the resonator is provided outside a laminated semiconductor layer, which is a main body of the excitation light source. As described later, the laser element according to the present disclosure has a structure in which the solid-state laser medium is arranged between the laminated semiconductor layer and the mirror arranged outside the laminated semiconductor layer.

As described above, the laser element according to the present disclosure can generate the laser pulse having a short pulse width by Q-switching, but the laser light is condensed inside the second resonator by a thermal lens effect generated with a rise in temperature of the solid-state laser medium. Inside the second resonator, since a peak intensity of the laser light becomes high due to pulse shortening, an optical damage inside the first resonator and the second resonator is likely to occur more remarkably due to the condensing of the laser light by the thermal lens effect. Especially, since the laminated semiconductor layer forming the first resonator includes a material having a small band gap, when leaked light of the laser light having the second wavelength generated in the second resonator enters the first resonator, the optical damage caused by multiphoton absorption is likely to occur.

Therefore, in the laser element according to the present disclosure, a peak condensing density of Q-switched laser pulses is decreased by providing an optical element that increases a beam diameter of the laser light having the second wavelength generated in the second resonator. Therefore, the laser element capable of avoiding the optical damage inside the first resonator and the second resonator without impairing advantages of a compact integrated structure, and a method of manufacturing the same are provided.

The laser element according to the present disclosure has following three features.
(1) The first resonator and the second resonator share the solid-state laser medium. The first resonator includes the excitation light source and the solid-state laser medium. The second resonator includes the solid-state laser medium and a saturable absorber, and performs Q-switched laser oscillation by excitation light from the first resonator.
(2) The optical element that increases the beam diameter of the laser light having the second wavelength generated by the second resonator is provided inside the second resonator. This optical element has a beam diverging action, that is, a negative refractivity function.
(3) The excitation light source, the solid-state laser medium, and the saturable absorber have an integrated structure.

In the laser element according to the present disclosure, the solid-state laser medium in the first resonator absorbs the excitation light generated by injecting a current into the excitation light source. The solid-state laser medium forms the second resonator together with the saturable absorber installed adjacent to the first resonator. When the solid-state laser medium is put into a sufficiently excited state, and an output of spontaneous emission light is increased to exceed a certain threshold, a light absorption rate in the saturable absorber rapidly decreases, and the spontaneous emission light generated in the solid-state laser medium can be transmitted through the saturable absorber, causing stimulated emission in the solid-state laser medium. This causes Q-switched pulse oscillation.

### (Basic Configuration of Laser Element)

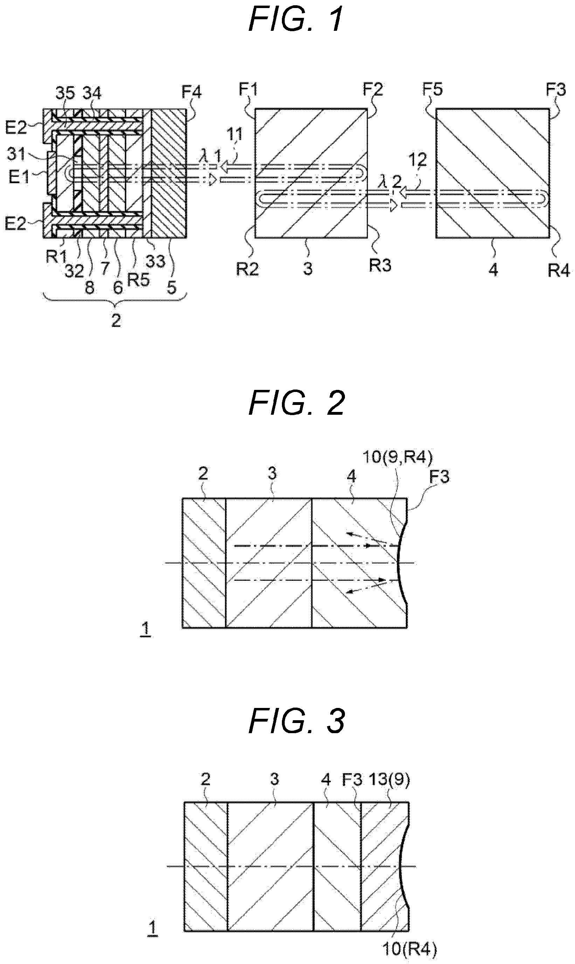

Hereinafter, a specific embodiment of the laser element according to the present disclosure is described. Fig. 1 is a diagram illustrating a basic configuration of a laser element 1 according to the present disclosure. The laser element 1 in Fig. 1 has a configuration in which an excitation light source 2, a solid-state laser medium 3, and a saturable absorber 4 are integrally joined.

The excitation light source 2 is a partial structure of the above-described VCSEL and includes the laminated semiconductor layer having a laminated structure. Hereinafter, the excitation light source 2 is sometimes referred to as a laminated semiconductor layer 2. The excitation light source 2 in Fig. 1 has a structure obtained by stacking a substrate 5, an n-contact layer 33, a fifth reflection layer R5, a cladding layer 6, an active layer 7, a cladding layer 8, a pre-oxidation layer 31, and a first reflection layer R1 in this order. Note that, the laser element 1 in Fig. 1 has a bottom emission type configuration in which continuous wave (CW) excitation light is emitted from the substrate 5, but may have a top emission type configuration in which the CW excitation light is emitted from the first reflection layer R1 side.

The substrate 5 is, for example, an n-GaAs substrate 5. Since the n-GaAs substrate 5 absorbs light having a first wavelength λ1, which is the excitation wavelength of the excitation light source 2, at a certain rate, this is desirably made as thin as possible. In contrast, it is desirable to provide such a thickness that can maintain mechanical strength at the time of a joining process to be described later.

The active layer 7 performs surface emission at the first wavelength λ1. The cladding layers 6 and 8 are, for example, AlGaAs cladding layers. The first reflection layer R1 reflects the light having the first wavelength λ1. The fifth reflection layer R5 has a certain transmittance with respect to the light having the first wavelength λ1. For the first reflection layer R1 and the fifth reflection layer R5, for example, a semiconductor distributed Bragg reflector (DBR) capable of performing electrical conduction is used. A current is externally injected via the first reflection layer R1 and the fifth reflection layer R5, recombination and light emission occur in a quantum well in the active layer 7, and laser oscillation at the first wavelength λ1 is performed. A part of the pre-oxidation layer (for example, AlAs layer) 31 on the cladding layer side of the first reflection layer R1 is oxidized to become a post-oxidation layer (for example, Al₂O₃ layer) 32.

The fifth reflection layer R5 is arranged on, for example, the n-GaAs substrate 5. For example, the fifth reflection layer R5 includes a multilayer reflection film containing Al_{z1}Ga_{1-z1}As/Al_{z2}Ga_{1-z2}As (0 ≤ z1 ≤ z2 ≤ 1) to which an n-type dopant (for example, silicon) is added. The fifth reflection layer R5 is also referred to as n-DBR. More specifically, the n-contact layer 33 is arranged between the fifth reflection layer R5 and the n-GaAs substrate 5.

The active layer 7 includes, for example, a multiple quantum well layer in which an Alₓ₁In_{y1}Ga_{1-x1-y1}As layer and an Alₓ₃In_{y3}Ga_{1-x3-y3}As layer are stacked.

The first reflection layer R1 includes a multilayer reflection film containing Alz3Ga1-z3As/Alz4Ga1-z4As (0 ≤ z3 ≤ z4 ≤ 1) to which a p-type dopant (for example, carbon) is added. The first reflection layer R1 is also referred to as p-DBR.

Each of the semiconductor layers R5, 6, 7, 8, and R1 in the excitation light source 2 can be formed by using a crystal growth method such as a metal organic chemical vapor deposition (MOCVD) method or a molecular beam epitaxy (MBE) method. Then, after the crystal growth, driving by current injection becomes possible after processes such as mesa etching for element separation, formation of an insulating film, and vapor deposition of an electrode film.

The solid-state laser medium 3 is joined to an end face on the side opposite to the fifth reflection layer R5 of the n-GaAs substrate 5 of the excitation light source 2. Hereinafter, an end face on the excitation light source 2 side of the solid-state laser medium 3 is referred to as a first surface F1, and an end face on the saturable absorber 4 side of the solid-state laser medium 3 is referred to as a second surface F2. Furthermore, a laser pulse emission surface of the saturable absorber 4 is referred to as a third surface F3, and an end face on the solid-state laser medium 3 side of the excitation light source 2 is referred to as a fourth surface F4. Furthermore, an end face on the solid-state laser medium 3 side of the saturable absorber 4 is referred to as a fifth surface F5. Although illustrated separately for convenience in Fig. 1, the fourth surface F4 of the excitation light source 2 is joined to the first surface F1 of the solid-state laser medium 3, and the second surface F2 of the solid-state laser medium 3 is joined to the fifth surface F5 of the saturable absorber 4.

The laser element 1 in Fig. 1 includes a first resonator 11 and a second resonator 12. The first resonator 11 causes the light having the first wavelength λ1 to resonate between the first reflection layer R1 in the excitation light source 2 and the third reflection layer R3 in the solid-state laser medium 3. The second resonator 12 causes the light having the second wavelength λ2 to resonate between the second reflection layer R2 in the solid-state laser medium 3 and the fourth reflection layer R4 in the saturable absorber 4.

The second resonator 12 is also referred to as a Q-switched solid-state laser resonator 12. A third reflection layer R3, which is a high reflection layer, is provided in the solid-state laser medium 3 so that the first resonator 11 can perform a stable resonance operation. In the normal excitation light source 2, a partial reflecting mirror as an output coupling mirror (output coupler) for emitting the light having the first wavelength λ1 to the outside is arranged at a position of the third reflection layer R3 in Fig. 1. In contrast, in the laser element 1 in Fig. 1, the high reflection layer is used as the third reflection layer R3 in order to use the third reflection layer R3 for confining power of the excitation light having the first wavelength λ1 in the first resonator 11.

In this manner, three reflection layers (first reflection layer R1, fifth reflection layer R5, and third reflection layer R3) are provided inside the first resonator 11 including the excitation light source 2 and the solid-state laser medium 3. Therefore, the first resonator 11 has a coupled resonator (coupled cavity) structure.

The solid-state laser medium 3 is excited by confining the power of the excitation light having the first wavelength λ1 in the first resonator 11. Therefore, Q-switched laser pulse oscillation occurs in the second resonator 12. The second resonator 12 causes the light having the second wavelength λ2 to resonate between the second reflection layer R2 in the solid-state laser medium 3 and the fourth reflection layer R4 in the saturable absorber 4. The second reflection layer R2 is the high reflection layer, whereas the fourth reflection layer R4 is a partial reflection layer, and plays a role of the output coupling mirror (output coupler). In Fig. 1, the fourth reflection layer R4 is provided on the end face of the saturable absorber 4, but the fourth reflection layer R4 may be arranged on a rear side of an optical axis with respect to the laser pulse emission surface of the saturable absorber 4. The rear side of the optical axis is in a light emission direction on the optical axis. That is, the fourth reflection layer R4 is not necessarily provided inside or on the surface of the saturable absorber 4. However, even in a case where the fourth reflection layer R4 is arranged on a front side of the optical axis with respect to the saturable absorber 4, it is necessary to cause the light having the second wavelength λ2 to resonate between the second reflection layer R2 and the fourth reflection layer R4.

The solid-state laser medium 3 contains, for example, ytterbium (Yb)-doped yttrium aluminum garnet (YAG) crystal Yb:YAG. In this case, the first wavelength λ1 of the first resonator 11 is 940 nm, and the second wavelength λ2 of the second resonator 12 is 1030 nm.

The solid-state laser medium 3 is not limited to Yb:YAG, and for example, at least any material of Nd:YAG, Nd:YVO4, Nd:YLF, Nd:glass, Yb:YAG, Yb:YLF, Yb:FAP, Yb:SFAP, Yb:YVO, Yb:glass, Yb:KYW, Yb:BCBF, Yb:YCOB, Yb:GdCOB, and YB:YAB can be used as the solid-state laser medium 3. A form of the solid-state laser medium 3 is not limited to a crystal, and the use of a ceramic material is not prevented.

Furthermore, the solid-state laser medium 3 may be a four-level system solid-state laser medium 3 or a quasi-three-level system solid-state laser medium 3. However, since an appropriate excitation wavelength (first wavelength λ1) varies depending on each crystal, it is necessary to select the semiconductor material of the active layer 7 in the excitation light source 2 according to the material of the solid-state laser medium 3.

The saturable absorber 4 contains, for example, a chromium (Cr)-doped YAG (Cr:YAG) crystal. The saturable absorber 4 is a material of which transmittance increases when intensity of incident light exceeds a predetermined threshold. The excitation light having the first wavelength λ1 by the first resonator 11 increases the transmittance of the saturable absorber 4 to emit the laser pulse having the second wavelength λ2. This is referred to as passive Q-switching. As a material of the saturable absorber 4, V:YAG can also be used. However, other types of saturable absorber 4 may also be used. Furthermore, the use of an active Q-switched element as the Q-switching is not prevented.

In Fig. 1, the excitation light source 2, the solid-state laser medium 3, and the saturable absorber 4 are separately illustrated, but they are joined to be integrated by using a joining process to form a laminated structure. Examples of the joining process include surface activation joining, atomic diffusion joining, plasma activation joining and the like. Alternatively, other joining (adhering) processes can be used.

In order to stably join the solid-state laser medium 3 to the excitation light source 2, it is necessary to flatten the surface of the n-GaAs substrate 5 in the excitation light source 2. Therefore, as described above, it is desirable that electrodes E1 and E2 for injecting a current into the first reflection layer R1 and the fifth reflection layer R5 be arranged so as not to be exposed at least on the surface of the n-GaAs substrate 5. In the example in Fig. 1, the electrodes E1 and E2 are arranged on the end face on the first reflection layer R1 side of the excitation light source 2. The electrode E1 is a p-electrode, and is electrically conducted with the first reflection layer R1. The electrode E2 is an n-electrode, and is formed by filling an inner wall of a trench reaching the n-contact layer 33 from the first reflection layer R1 with a conductive material 35 via an insulating film 34. By arranging the electrodes E1 and E2 on the same end face of the excitation light source 2 as in Fig. 1, this end face can be soldered to a support substrate not illustrated. Also when a plurality of laser elements is arranged in an array, by arranging the electrodes E1 and E2 on the same end face, this end face can be mounted on the support substrate. Note that, shapes and arranged positions of the electrodes E1 and E2 illustrated in Fig. 1 are illustrative only.

In this manner, by forming the laser element 1 in Fig. 1 into a laminated structure, it becomes easy to form a plurality of chips by dicing the laminated structure to separate after fabricating the same, or to form a laser array in which a plurality of laser elements 1 is arranged in an array on one substrate.

In a case where the laser element 1 having the laminated structure is fabricated by the joining process, arithmetic average roughness Ra of each surface layer needs to be about 1 nm or less, and is desirably 0.5 nm or less. Chemical mechanical polishing (CMP) is used to implement the surface layer having such arithmetic average roughness. Furthermore, in order to avoid an optical loss at an interface of each layer, a dielectric multilayer film may be arranged between the layers, and the layers may be joined via the dielectric multilayer film. For example, the GaAs substrate 5 as a base substrate of the excitation light source 2 has a refractive index n of 3.2 with respect to a wavelength of 940 nm, which is higher than that of YAG (n:1.7) or a general dielectric multilayer film material. Therefore, when the solid-state laser medium 3 and the saturable absorber 4 are joined to the excitation light source 2, it is necessary to prevent the optical loss due to refractive index mismatch from occurring. Specifically, it is desirable to arrange an anti-reflection film (AR coating film or non-reflection coating film) that does not reflect the light having the first wavelength λ1 of the first resonator 11 between the excitation light source 2 and the solid-state laser medium 3. Furthermore, it is desirable to arrange an anti-reflection film (AR coating film or non-reflection coating film) also between the solid-state laser medium 3 and the saturable absorber 4.

Polishing is sometimes difficult depending on a joining material, and for example, it is possible to deposit a material transparent with respect to the first wavelength λ1 and the second wavelength λ2 such as SiO₂ as a base layer for joining, and polish this SiO₂ layer to have arithmetic average roughness Ra of about 1 nm (preferably 0.5 nm or less) to use as an interface for joining. Here, a material other than SiO₂ can be used as the base layer, and the material is not limited. Note that, a non-reflection film may be provided between SiO₂ as the material of the base layer and a base material layer.

Examples of the dielectric multilayer film include a short wave pass filter (SWPF), a long wave pass filter (LWPF), a band pass filter (BPF), an anti-reflection (AR) protective film and the like. It is desirable to arrange different types of dielectric multilayer films as necessary. A physical vapor deposition (PVD) method can be used as a film deposition method of the dielectric multilayer film, and specifically, the film deposition method such as vacuum vapor deposition, ion-assisted vapor deposition, and sputtering can be used. It does not matter which film deposition method is applied. Furthermore, any characteristic of the dielectric multilayer film can be selected, and for example, the second reflection layer R2 may be the short wave pass filter, and the third reflection layer R3 may be the long wave pass filter. By applying the short wave pass filter to the second reflection layer R2, it is possible to prevent pulse light having the second wavelength from entering the semiconductor layer and to prevent damage to the semiconductor.

Furthermore, by applying the long wave pass filter to the third reflection layer R3, it is possible to prevent the first wavelength from entering the saturable absorber and to prevent malfunction of the Q-switching. Note that, the short wave pass means that the light having the first wavelength λ1 is transmitted and the light having the second wavelength λ2 is reflected. Furthermore, the long wave pass means that the light having the first wavelength λ1 is reflected and the light having the second wavelength λ2 is transmitted.

Furthermore, a polarizer having a photonic crystal structure that separates a ratio of P-polarized light and S-polarized light may be provided inside the second resonator 12. Furthermore, it is possible to provide a diffraction grating inside the second resonator 12 to convert a polarization state of the emitted laser pulse from random polarization to linear polarization.

### (Operation Principal of Laser Element 1)

Next, an operation of the laser element 1 in Fig. 1 is described. By injecting a current into the active layer 7 via the electrode of the excitation light source 2, laser oscillation at the first wavelength λ1 occurs in the first resonator 11, and the solid-state laser medium 3 is excited. Since the saturable absorber 4 is joined to the solid-state laser medium 3, in an initial stage when the laser oscillation at the first wavelength λ1 occurs, the spontaneous emission light from the solid-state laser medium 3 is absorbed by the saturable absorber 4, so that optical feedback by the fourth reflection layer R4 on the emission surface side of the saturable absorber 4 does not occur, and the Q-switched laser oscillation does not occur.

Thereafter, when the power of the excitation light having the first wavelength λ1 is accumulated in the solid-state laser medium 3, and the solid-state laser medium 3 is put into a sufficiently excited state, an output of the spontaneous emission light is increased, and when this exceeds a certain threshold, a light absorption rate in the saturable absorber 4 rapidly decreases, and the spontaneous emission light generated in the solid-state laser medium 3 can be transmitted through the saturable absorber 4. Therefore, the light having the first wavelength λ1 by the first resonator 11 is emitted from the solid-state laser medium 3, and the second resonator 12 causes the light having the second wavelength λ2 to resonate between the second reflection layer R2 and the fourth reflection layer R4. Therefore, the Q-switched laser oscillation occurs, and the Q-switched laser pulse is emitted toward a space (space on a right side in Fig. 1) via the fourth reflection layer R4.

A non-linear optical crystal for wavelength conversion can be arranged inside the second resonator 12. The wavelength of the laser pulse after the wavelength conversion can be changed depending on a type of the non-linear optical crystal. Examples of wavelength converting materials include non-linear optical crystals such as LiNbO₃, BBO, LBO, CLBO, BiBO, KTP, and SLT. Furthermore, a phase-matching material similar to them may be used as the wavelength converting material. Note that, the type of the wavelength converting material is not limited. The second wavelength λ2 can be converted to another wavelength by the wavelength converting material.

Although the basic configuration and the operation principle for obtaining the Q-switched laser oscillation according to the present disclosure are described above, the above-described configuration cannot solve a problem of the optical damage of the resonator material (including the dielectric multilayer film) due to the high-peak pulse that is shortened as described at the beginning.

### (Solution to Optical Damage)

First, a cause of the optical damage is described. A part of energy of the excitation light absorbed by the solid-state laser medium 3 in Fig. 1 is converted into heat, and temperature of the solid-state laser medium 3 rises. At that time, a temperature distribution is generated, and a refractive index distribution associated with the temperature distribution is generated. In general, in many solid-state laser media 3 including an inorganic material, the higher the temperature, the higher the refractive index.

Since the temperature is higher in the optical axis center portion where the intensity of the excitation light is higher, the refractive index in the vicinity of the optical axis center is higher, and the refractive index in a peripheral portion becomes gradually lower as it is farther from the optical axis. In this manner, a virtual condenser lens referred to as a thermal lens is generated in the solid-state laser medium 3. In the present disclosure, since a resonator length of the second resonator 12 can be shortened by making a compact integrated laser element 1, the pulse width of the laser pulse emitted from the laser element 1 becomes further shorter. The shorter the pulse width of the laser pulse, the higher the peak power, so that the optical damage is more likely to occur than in the conventional case. Especially, the optical damage of the dielectric multilayer film forming the interface is likely to occur.

The optical damage occurs not only inside the second resonator 12 that generates the Q-switched laser pulse but also inside the excitation light source 2 (excitation light source 2) because return light is generated on the excitation light source 2 side. Especially, since the semiconductor layer 2 having the laminated structure forming the excitation light source 2 is formed by using a material having a small band gap, the optical damage caused by multiphoton absorption by short-pulse laser light is likely to occur. Note that, also in a case where a laser pulse is generated by a semiconductor laser alone, the optical damage caused by the multiphoton absorption by the short-pulse laser light occurs, so that it is difficult to increase the peak power of the laser pulse.

In order to solve this problem, it is sufficient to increase the beam diameter of the light having the second wavelength λ2 inside the second resonator 12. For this purpose, it is sufficient to provide an optical element that enlarges the beam diameter of the light having the second wavelength λ2 inside the second resonator 12. The optical element reflects or refracts at least a part of the light having the second wavelength λ2 so that the light having the second wavelength λ2 is not condensed. That is, the optical element has a light diverging action or a negative refracting action. The optical element includes, for example, a convex mirror that reflects at least a part of the incident light or a light refracting member that refracts at least a part of the incident light so that the incident light is not condensed. Since the light incident on the convex mirror is reflected so as not to be condensed or the light incident on the light refracting member is refracted so as not to be condensed, the beam diameter of the light having the second wavelength λ2 can be enlarged. The optical element is arranged between the second reflection layer R2 and the fourth reflection layer R4 in the second resonator 12. For example, the optical element is arranged inside or on the surface of at least one of the solid-state laser medium 3 or the saturable absorber 4 arranged between the second reflection layer R2 and the fourth reflection layer R4. In a case where the optical element includes the fourth reflection layer, the fourth reflection layer is the convex mirror, and in a case where the optical element is provided on a side closer to the second reflection layer than the fourth reflection layer, the optical element includes the light refracting member. Hereinafter, various modes of the above-described optical element are described.

### (First Mode of Optical Element)

Fig. 2 is a schematic cross-sectional view illustrating a first mode of the optical element 9. Fig. 2 illustrates a cross-sectional structure of the laser element 1 including the excitation light source 2, the solid-state laser medium 3 formed by using an ytterbium (Yb)-doped yttrium aluminum garnet (YAG) crystal as a material, for example, and the saturable absorber 4 formed by using a chromium (Cr)-based YAG crystal as a material.

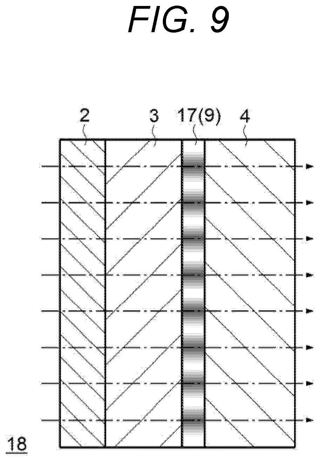

The optical element 9 in Fig. 2 is arranged along the laser pulse emission surface (third surface F3) of the saturable absorber 4. The optical element 9 in Fig. 2 is obtained by processing the third surface F3 of the saturable absorber 4 into a concave shape. More specifically, the optical element 9 in Fig. 2 includes the convex mirror 10 obtained by processing the third surface F3 into the concave shape, and forming the dielectric multilayer film or the like along the processed concave surface. The convex mirror 10 also serves as the fourth reflection layer R4 that reflects the light having the second wavelength λ2 resonated between the laser medium 3 and the saturable absorber 4. Note that, although the electrodes E1 and E2 illustrated in Fig. 1 are omitted in Fig. 2 and Figs. 3 to 9 described later, the excitation light source 2 includes the electrodes E1 and E2 similar to those in Fig. 1.

As indicated by a dashed-dotted line in Fig. 2, the convex mirror 10 in the optical element 9 in Fig. 2 reflects the light having the second wavelength λ2 generated in the second resonator 12 in such a manner that this is not condensed. Therefore, the beam diameter of the light having the second wavelength λ2 in the second resonator 12 can be increased, and the peak power of the laser light can be reduced, so that the optical damage is less likely to occur in the solid-state laser medium 3 and the excitation light source 2.

The optical element 9 in Fig. 2 can be relatively easily formed by processing the third surface F3 of the saturable absorber 4 by etching, polishing and the like. Furthermore, a curvature of the convex mirror 10 can be adjusted to any curvature by adjusting an etching material, an etching time, a polishing time and the like. The beam diameter of the light beam having the second wavelength λ2 can be controlled by adjusting the curvature of the convex mirror 10. The curvature of the convex mirror 10 depends on the power of the excitation light having the first wavelength λ1 in the first resonator 11, the resonator length of the second resonator 12, the refractive index of the base substrate of the optical element 9 and the like.

Although Fig. 2 illustrates an example in which the optical element 9 is arranged along the third surface F3 of the saturable absorber 4, the optical element 9 can be provided at any place between the second reflection surface R2 and the fourth reflection surface R4 in Fig. 1. For example, the optical element 9 having a concave shape may be provided along the first surface F1 in the solid-state laser medium 3.

In this manner, the beam diameter of the light having the second wavelength λ2 in the second resonator 12 can be increased by providing the optical element 9 along the end face of the saturable absorber 4 or the solid-state laser medium 3 or inside the saturable absorber 4 or the solid-state laser medium 3. The optical element 9 in Fig. 2 is obtained by processing the end face of the saturable absorber 4 and the solid-state laser medium 3 by etching, polishing and the like to form the convex mirror 10 of the dielectric multilayer film and the like, and is easily manufactured. Furthermore, the beam diameter of the light having the second wavelength λ2 can be relatively easily controlled by adjusting the curvature at the time of concave surface processing.

### (Second Mode of Optical Element 9)

Fig. 3 is a schematic cross-sectional view illustrating a second mode of the optical element 9. The optical element 9 in Fig. 3 is provided separately from the solid-state laser medium 3 and the saturable absorber 4, and is joined to the third surface F3 of the saturable absorber 4. The optical element 9 in Fig. 3 is formed by using a material that transmits the light having the second wavelength λ2, and is formed by using a transparent medium 13 that is transparent with respect to the light having the second wavelength λ2 as a material. The optical element 9 in Fig. 3 is desirably formed by using a material having high thermal conductivity. Examples of a candidate for the transparent medium 13, which is a base material of the optical element 9 in Fig. 3, include sapphire, silicon carbide (SiC), and chemical vapor deposition (CVD) diamond and the like, for example.

As in Fig. 2, the fourth reflection layer R4 including the convex mirror 10 is provided on the laser pulse emission surface of the optical element 9 in Fig. 3. In this manner, in the laser element 1 in Fig. 3, the fourth reflection layer R4 is provided not on the third surface F3, which is the end face of the saturable absorber 4, but on the end face of the optical element 9 arranged on the rear side of the optical axis with respect to the saturable absorber 4.

The end face of the optical element 9 in Fig. 3 is processed into a concave shape, and a reflection film such as a dielectric multilayer film is formed on the processed surface to form the convex mirror 10. As the material of the optical element 9 in Fig. 3, a material not only having high thermal conductivity but also excellent in processability is desirable. From this viewpoint, sapphire is desirable among the material candidates described above. Note that, the material of the optical element 9 in Fig. 3 may be any specific material as long as the material has high thermal conductivity and excellent processability.

Furthermore, although Fig. 3 illustrates an example in which the optical element 9 is joined to the third surface F3 of the saturable absorber 4, the optical element 9 in Fig. 3 may be arranged between the saturable absorber 4 and the solid-state laser medium 3 or between the solid-state laser medium 3 and the excitation light source 2.

In this manner, since the optical element 9 is provided separately from the solid-state laser medium 3 and the saturable absorber 4 to be joined to the saturable absorber 4 and the solid-state laser medium 3, the laser element 1 having an integrated structure including the optical element 9 can be fabricated. In the laser element 1 in Fig. 3, since it is not necessary to process the shapes of the solid-state laser medium 3 and the saturable absorber 4, there is no possibility of adversely affecting the emission of the laser pulse.

### (Third Mode of Optical Element 9)

In the laser element 1 in Fig. 1, the excitation light source 2, the solid-state laser medium 3, and the saturable absorber 4 are joined to form the integrated structure. When the end face of the optical element 9 is processed into the concave shape as in Figs. 2 and 3, there is a possibility that a gap is generated in a joint portion between the solid-state laser medium 3 or the saturable absorber 4 provided with the optical element 9 and another member as is. Therefore, in the third mode of the optical element 9, the surface of the optical element 9 is processed into a concave shape and a reflection film such as a dielectric multilayer film is formed, and thereafter the surface of the optical element 9 including the concave surface is flattened.

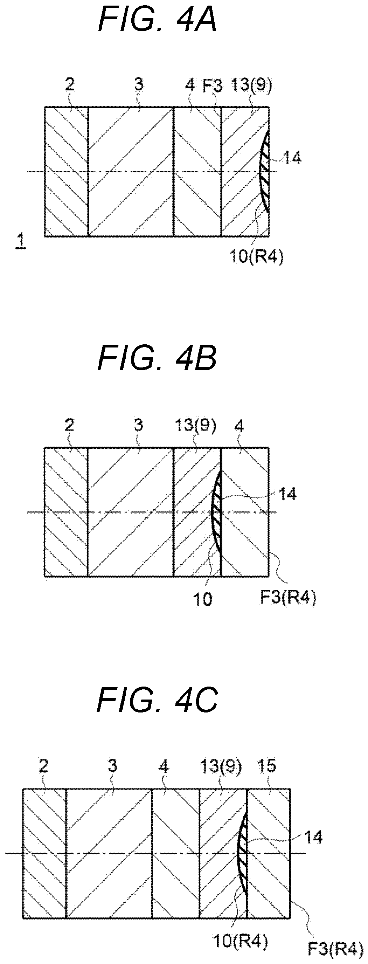

As a material for flattening the surface of the optical element 9, a material that is transparent with respect to the light having the second wavelength λ2 and has a refractive index smaller than the refractive index of the optical element 9 is used. Figs. 4A, 4B, and 4C are schematic cross-sectional views illustrating three representative examples of the third mode of the optical element 9. As in Fig. 3, in Fig. 4A, the optical element 9 joined to the third surface F3 of the saturable absorber 4 is provided. The laser pulse emission surface of the optical element 9 is processed into a concave shape, and a flattening member 14 is arranged along the concave surface. As described above, as the flattening member 14, a material transparent with respect to the light having the second wavelength λ2, an insulating material having a smaller refractive index than that of sapphire, such as SiO₂, for example, is used. The flattening member 14 also serves as the light refracting member 14.

In Fig. 4B, the optical element 9 arranged between the solid-state laser medium 3 and the saturable absorber 4 is provided, and a joint surface to the saturable absorber 4 of the optical element 9 is processed into a concave shape to form the light refracting member 14. A surface on the side opposite to a concave surface 14a of the light refracting member 14 is a flat surface, and is joined to the saturable absorber 4 on an entire end face of the optical element 9 including the flat surface. Therefore, a joining property between the optical element 9 and the saturable absorber 4 is improved. The light refracting member 14 in Fig. 4B refracts the incident light and causes the same to be incident on the saturable absorber 4 so that the incident light is not condensed. The light incident on the saturable absorber 4 is reflected by the fourth reflection layer R4, which is the end face of the saturable absorber 4.

Fig. 4C is a variation of Fig. 4A in which a photonic crystal layer 15 is joined to the flattened surface of the light refracting member 14 including the concave surface of the optical element 9. The light refracting member 14 in Fig. 4C refracts the incident light and causes the same to be incident on the photonic crystal layer 15 so that the incident light is not condensed. The photonic crystal layer 15 is a layer of which refractive index periodically changes. Note that, the photonic crystal layer 15 is an example, and various light control members that reflect or refract the incident light from the optical element 9 can be joined to the optical element 9.

In this manner, as illustrated in Figs. 4A, 4B, and 4C, in the third mode of the optical element 9, since the end face of the convex mirror 10 or the light refracting member 14 in the optical element 9 is made flat, the joining property with another member is improved, and the laser element 1 having an integrated structure can be obtained.

### (Fourth Mode of Optical Element 9)

In the first to third modes described above, the example in which the end face of the optical element 9 is processed into the concave shape to form the convex mirror 10 or the light refracting member 14 is described; however, also in a case where the end surface of the optical element 9 has a fine periodic structure, the beam diameter of the second wavelength λ2 can be increased.

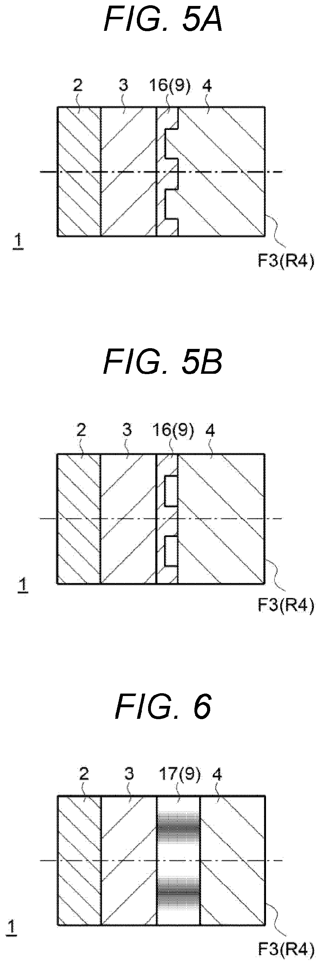

Figs. 5A and 5B are schematic cross-sectional views illustrating a fourth mode of the optical element 9. Fig. 5A illustrates an example in which the optical element 9 including a fine periodic structure 16 is provided along the joint surface to the solid-state laser medium 3 of the saturable absorber 4. The fine periodic structure 16 in Fig. 5A is formed, for example, by processing the fifth surface F5 of the solid-state laser medium 3 to provide periodic irregularities.

Fig. 5B illustrates an example in which the optical element 9 having the fine periodic structure 16 is provided between the saturable absorber 4 and the solid-state laser medium 3. The fine periodic structure 16 in Fig. 5B is a member separate from the saturable absorber 4 and the solid-state laser medium 3, and is joined to the saturable absorber 4 and the solid-state laser medium 3.

Note that, the optical element 9 having the fine periodic structure 16 may be provided along the third surface F3, which is the end face of the saturable absorber 4, or may be provided in the solid-state laser medium 3.

More specifically, the fine periodic structure 16 in Figs. 5A and 5B is, for example, a Fresnel lens, a meta lens, or a photonic crystal lens. The beam diameter of the light having the second wavelength λ2 can be adjusted by adjusting the period and size of the irregularities forming the fine periodic structure 16.

The fine periodic structure 16 in Fig. 5 can be relatively easily formed by using a semiconductor process technology such as photolithography or imprinting, for example.

In this manner, in the fourth mode of the optical element 9, since the beam diameter of the light having the second wavelength λ2 is increased by the fine periodic structure 16, it is possible to avoid the optical damage due to the beam condensing inside the second resonator 12 as is the case with the first to third modes.

### (Fifth Mode of Optical Element 9)

In a fifth mode of the optical element 9, a refractive index distribution is provided inside the optical element 9. The optical element 9 having the refractive index distribution is also referred to as a gradient index (GRIN) lens 17.

Fig. 6 is a schematic cross-sectional view illustrating a fifth mode of the optical element 9. Fig. 6 illustrates an example in which the optical element 9 including the GRIN lens 17 is arranged between the solid-state laser medium 3 and the saturable absorber 4. Note that, the optical element 9 including the GRIN lens 17 may be provided along the third surface F3, which is the end face of the saturable absorber 4, or may be provided in the solid-state laser medium 3.

In the fifth mode of the optical element 9, since the surface is flat, the joining property to another member is improved. Furthermore, since the optical element 9 including the photonic crystal can have any refractive index distribution using a semiconductor process technology, the beam diameter of the light having the second wavelength λ2 can be adjusted to any diameter.

### (Sixth Mode of Optical Element 9)

In the first to fifth modes of the optical element 9, the single laser element 1 is described, but it is also applicable to a laser array 18 in which a plurality of laser elements 1 is arranged in an array in a one-dimensional direction or a two-dimensional direction.

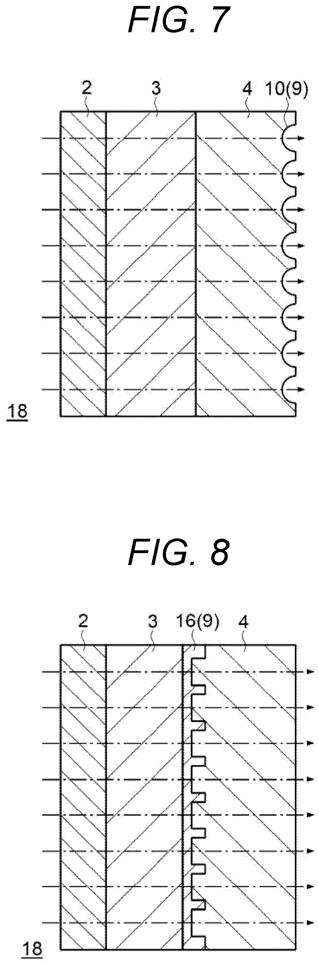

Figs. 7, 8, and 9 are schematic cross-sectional views of the laser array 18 in which the laser elements 1 in Figs. 3, 5, and 6 are arranged in an array. When the excitation light source 2, the solid-state laser medium 3, and the saturable absorber 4 are joined, by providing an alignment mark at a specific site of each of the excitation light source 2, the solid-state laser medium 3, and the saturable absorber 4, and aligning in such a manner that these alignment marks overlap to join, the optical axis of the excitation light source 2, the optical axis of the solid-state laser medium 3, and the optical axis of the saturable absorber 4 can be coaxially aligned. Furthermore, even in a case where the optical element 9 is separately provided, it is possible to provide an alignment mark at a specific site of the optical element 9 to coaxially align the optical axis of the optical element 9 with the optical axis of the solid-state laser medium 3 or the saturable absorber 4.

In this manner, by joining the excitation light source 2, the solid-state laser medium 3, and the saturable absorber 4 by aligning using the alignment marks, the optical axes of the laser elements 1 forming the laser array 18 can be aligned at once, and cost reduction and a compact size due to simplification of the manufacturing process can be implemented.

### (Heat Exhausting Function)

When the excitation light source 2 and the solid-state laser medium 3 are joined and the first resonator 11 generates the excitation light having the first wavelength λ1, in addition to heat generated from the excitation light source 2, heat by the power of the excitation light accumulated in the solid-state laser medium 3 is transmitted to the excitation light source 2, and an I-L characteristic (injection current-light output characteristic) of the excitation light source 2 is deteriorated. When the I-L characteristic is deteriorated, light emission efficiency of the excitation light source 2 is deteriorated. In contrast, when temperature of the solid-state laser medium 3 rises due to excitation light absorption, heat is transferred from the solid-state laser medium 3 to the excitation light source 2, and the temperature of the excitation light source 2 further rises. As a result, thermal interference between the excitation light source 2 and the solid-state laser medium 3 occurs, the I-L characteristic (light emission efficiency) of the excitation light source 2 is deteriorated, junction temperature Tj of the active layer 7 rises, and long-term reliability (mean time to failure: MTTF) is deteriorated.

Therefore, it is desirable to arrange at least one member that is transparent with respect to the light having the first wavelength λ1 and the light having the second wavelength λ2 and has a heat exhausting function in the laser element 1. Hereinafter, such member is referred to as a heat exhausting member. The heat exhausting member is, for example, the sapphire substrate 5 that has a refractive index and a linear expansion coefficient equivalent to those of YAG and is superior in thermal conductivity to YAG.

As described above, especially, since the thermal interference occurs between the excitation light source 2 and the solid-state laser medium 3, it is desirable that the heat exhausting member is arranged between the excitation light source 2 and the solid-state laser medium 3, and the excitation light source 2, the heat exhausting member, and the solid-state laser medium 3 have a laminated structure. This makes it possible to suppress deterioration in laser oscillation efficiency due to thermal interference without impairing advantages of the compact integrated structure.

### (Countermeasure against Return Light)

Furthermore, when the second resonator 12 emits the laser pulse having the second wavelength λ2 by Q-switching, there is a possibility that oscillation light having the second wavelength λ2 with high peak intensity enters the excitation light source 2 as return light. Since the excitation light source 2 is formed by using a semiconductor material having a small band gap, this might be broken by the return light. Therefore, it is desirable to arrange a plurality of short wave pass filter (SWPF) between the excitation light source 2 and the solid-state laser medium 3 while shortening the resonator length, thereby preventing the return light from entering the excitation light source 2.

### (Method of Manufacturing Laser Element 1 of Present Disclosure)

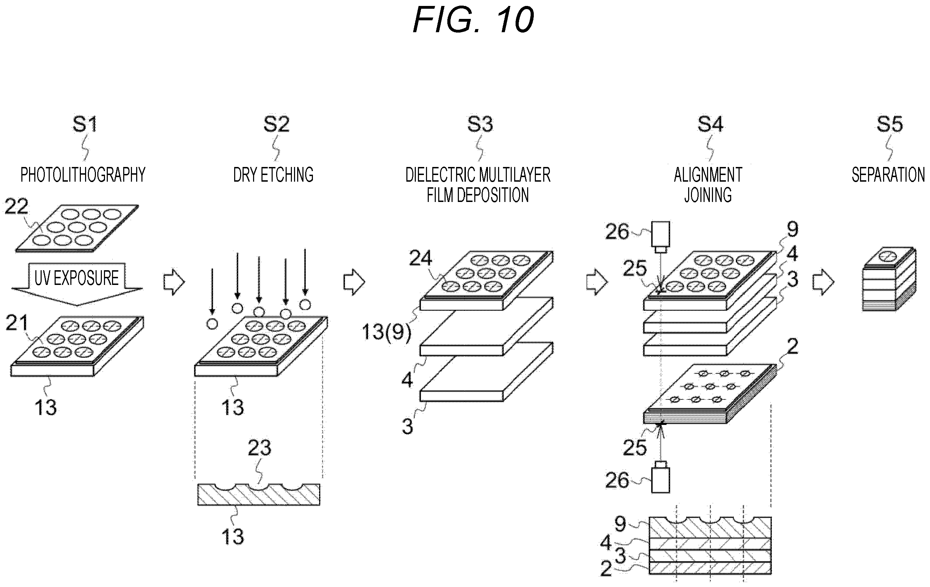

Fig. 10 is a diagram schematically illustrating a manufacturing process of the laser element 1 of the present disclosure. Fig. 10 illustrates the manufacturing process of forming the laser element 1 having the structure in Fig. 2. First, as illustrated at step S1 in Fig. 10, a resist film 21 is applied onto the third surface of the saturable absorber 4, a photomask 22 is arranged on the resist film 21, and UV exposure is performed.

Next, as illustrated at step S2, the exposed site and the resist film 21 are removed by dry etching and the like to form a plurality of concave portions 23 on the third surface of the saturable absorber 4. In the plurality of concave portions 23, a dielectric multilayer film 24 is formed by vapor deposition, sputtering and the like to form the convex mirror 10.

Next, as illustrated at step S3, the semiconductor layer 2 for the excitation light source 2, the solid-state laser medium 3, and the saturable absorber 4 processed at step S2 are arranged in the vertical direction to be aligned. At that time, as illustrated at step S4, an alignment mark 25 provided at a specific site of each of the semiconductor layer 2, the solid-state laser medium 3, and the saturable absorber 4 is imaged by a camera 26, and the semiconductor layer 2, the solid-state laser medium 3, and the saturable absorber 4 are aligned in such a manner that the alignment marks 25 are vertically overlapped with each other to be joined. Next, as illustrated at step S5, this is separated into individual laser elements by dicing.

### (Effect of Laser Element 1 of Present Disclosure)

As described above, in this embodiment, the optical element 9 is provided to increase the beam diameter of the light having the second wavelength λ2 in the second resonator 12 for the possibility of the occurrence of the optical damage due to the shortening of the laser pulse emitted from the saturable absorber 4. Therefore, the light having the second wavelength λ2 in the second resonator 12 is not condensed in the optical axis direction, and the optical damage in the solid-state laser medium 3 and the excitation light source 2 can be suppressed. Since the optical element 9 can be relatively easily formed by using a semiconductor process technology such as processing the third surface F3 of the saturable absorber 4 into a concave shape, it is possible to prevent the optical damage of the laser element 1 without impairing mass productivity, a compact size, and cost reduction.

In the laser element 1 according to the present disclosure, the solid-state laser medium 3 is shared by the first resonator 11 and the second resonator 12. Furthermore, light transmission surfaces of all the optical components including the excitation light source 2, the solid-state laser medium 3, and the saturable absorber 4 in the laser element 1 are joined and fixed by a joining process. Moreover, by providing the optical element 9 that increases the beam diameter of the Q-switched laser light inside the solid-state laser medium 3 and the saturable absorber 4, it is possible to suppress the optical damage due to the thermal lens effect. Therefore, reliability and mass productivity of the laser element 1 are improved, and the laser element 1 having high performance can be obtained at low cost.

According to the present disclosure, since the solid-state laser medium 3 is joined to the excitation light source 2, the solid-state laser medium 3 is excited by a standing wave in the excitation light source 2. By designing a resonator that confines the excitation light in the first resonator 11, even in a case where the solid-state laser medium 3 only has a thickness that cannot absorb the excitation light when the laser light passes through the first resonator 11 once, the excitation light can be sufficiently absorbed by the solid-state laser medium 3 finally when the laser light reciprocates a plurality of times. This makes it possible to perform the Q-switched laser oscillation with a shorter pulse without deterioration in excitation efficiency.

In the conventional Q-switched solid-state laser, the solid-state laser medium 3 is excited by a traveling wave, and the manner of exciting is significantly different from that of the laser element 1 according to the present disclosure. In the laser element 1 according to the present disclosure, the above-described trade-off that the absorption amount of the excitation light decreases when the solid-state laser medium 3 is shortened can be solved.

Furthermore, according to the laser element 1 of the present disclosure, it is possible to suppress short-term and long-term fluctuations in laser output due to mechanical positional deviation by directly joining the light transmission surfaces of the optical components. Furthermore, it is possible to join all the optical components and then dice the same to separate into individual laser light sources, mass productivity can be improved.

Conventionally, the excitation light source 2 and the second resonator 12 perform five-axis optical adjustment (X, Y, Z, θ, ϕ) with respect to the optical axis, eccentricity, and focus using a plurality of lenses including a collimator lens and a condenser lens. Furthermore, when it is attempted to add the optical element 9 having a beam diverging function (negative refractive power) to the second resonator 12, it becomes more difficult to accurately adjust the position of the optical element 9.

However, in the laser element 1 according to the present disclosure, in order to align a light emission point of the excitation light source 2 and the center position of the convex mirror 10 of the optical element 9 without using the plurality of lenses, which are the collimator lens and the condenser lens, joining is performed by using the alignment mark 25 and the like, so that it is not necessary to adjust focus position accuracy in a thickness (Z-axis) direction or inclination in θ and ϕ directions. Therefore, according to the laser element 1 of the present disclosure, it becomes possible to suppress the short-term and long-term fluctuations in laser output, optical adjustment becomes easy because the oscillation light is obtained from the excitation light source 2, and the light source with improved mass productivity can be implemented.

Furthermore, in the laser element according to the present disclosure, since the optical element 9 having a diverging action is included inside the second resonator 12 or on the end face thereof, it is possible to avoid the optical damage due to the beam condensing inside the second resonator 12.

Furthermore, the laser element 1 according to the present disclosure adopts a laminated structure in which the optical axis of the first resonator 11 and the optical axis of the second resonator 12 are integrated so as to be coaxial. In the laser element 1 according to the present disclosure, it is not necessary to perform complicated position and angle alignment, and the structure is simplified. Therefore, the laser element 1 can be easily made compact.

Furthermore, a plurality of laser elements 1 according to the present disclosure can be simultaneously formed by stacking or joining a plurality of materials on the same semiconductor substrate 5. By dicing in a post-process after the plurality of laser elements 1 is simultaneously formed to separate each laser element 1, it is possible to mass-produce high-performance laser elements 1 at a low cost. Furthermore, by the laser element 1 according to the present disclosure, the laser array 18 in which the plurality of laser elements 1 is two-dimensionally arranged on one substrate can also be easily fabricated.

Furthermore, in the laser element 1 according to the present disclosure, a repetition frequency of the laser pulse can be adjusted depending on the type of the solid-state laser medium 3. Especially, since the laser element 1 according to the present disclosure has a high gain density, the repetition frequency of the laser pulse can be increased. Furthermore, in the laser element 1 according to the present disclosure, the resonator length can be changed only by adjusting the thicknesses of the solid-state laser medium 3, the Q-switch (the saturable absorber 4), and the wavelength converting material (the non-linear optical crystal). That is, since the pulse time width of the laser pulse can be changed depending on the thickness of the material, the characteristic of the laser pulse can be easily adjusted. Especially, by shortening the pulse time width of the laser pulse, processing accuracy in the field of fine processing can be increased.

Moreover, by arraying the laser elements 1 according to the present disclosure in a one-dimensional array or a two-dimensional array, it is possible to obtain a laser device that achieves both high processing accuracy and high output energy. Furthermore, the laser element 1 according to the present disclosure can be applied to other fields such as highly efficient wavelength conversion technology, medical equipment, and ranging.

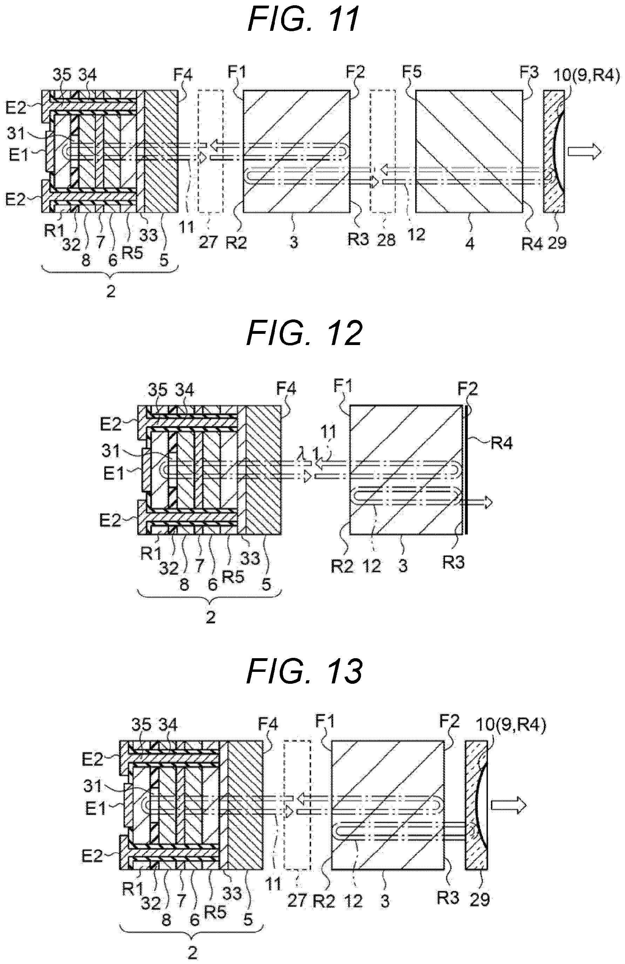

In the laser element 1 in Fig. 1, the example in which the excitation light source 2, the solid-state laser medium 3, and the saturable absorber 4 are integrally joined is illustrated; however, as illustrated in Fig. 11, a first transparent medium 27 that transmits the light having the first wavelength λ1 may be arranged between the excitation light source 2 and the solid-state laser medium 3.

Furthermore, as illustrated in Fig. 11, a second transparent medium 28 that transmits the light having the second wavelength λ2 may be arranged between the solid-state laser medium 3 and the saturable absorber 4. Note that, it is possible that only one of the first transparent medium 27 and the second transparent medium 28 is arranged.

In this manner, the excitation light source 2, the solid-state laser medium 3, and the saturable absorber 4 are not necessarily integrally joined.

Furthermore, as illustrated in Fig. 11, the optical element 9 may be arranged on the rear side of the optical axis with respect to the saturable absorber 4. In the optical element 9 in Fig. 11, a transparent member 29 that transmits the light having the second wavelength is processed into a concave shape to form the convex mirror 10. In a case in Fig. 11, the optical axis of the excitation light source 2, the optical axis of the solid-state laser medium 3, the optical axis of the saturable absorber 4, and the optical axis of the optical element 9 need to be coaxially arranged.

### (Laser Element without Saturable Absorber)

Fig. 1 illustrates an example in which the laser element 1 includes the saturable absorber 4 and emits the short-pulse laser light, but there is a possibility that the optical damage occurs also in the laser element 1 not including the saturable absorber 4 that emits CW laser light.

Fig. 12 is a diagram illustrating a basic configuration of the laser element 1 not including the saturable absorber 4. The laser element 1 in Fig. 12 has a configuration in which the saturable absorber 4 is omitted from Fig. 1. The first resonator 11 causes the light having the first wavelength λ1 to resonate between the first reflection layer R1 in the excitation light source 2 and the third reflection layer R3 in the solid-state laser medium 3 as in Fig. 1. In contrast, unlike in Fig. 1, the second resonator 12 causes the light having the second wavelength λ2 to resonate between the second reflection layer R2 and the fourth reflection layer R4 in the solid-state laser medium 3. The fourth reflection layer R4 is arranged on the second surface of the solid-state laser element 1 or on the rear side of the optical axis with respect to the second surface.

Fig. 12 illustrates an example in which the third reflection layer R3 and the fourth reflection layer R4 are separately provided along the second surface F2 of the solid-state laser medium 3. In a case where the fourth reflection layer R4 is arrange don the rear side of the optical axis with respect to the third reflection layer R3 as in Fig. 12, the third reflection layer R3 needs to have a characteristic of transmitting the light having the second wavelength.

The third reflection layer R3 is a high reflection layer with respect to the first wavelength, whereas the fourth reflection layer R4 is a partial reflection layer with respect to the second wavelength. Therefore, the power of the excitation light having the first wavelength is confined in the solid-state laser medium 3, and when the solid-state laser medium 3 is put into a sufficiently excited state and the output of the spontaneous emission light is increased, the light having the second wavelength is transmitted through the fourth reflection layer R4 to be emitted from the laser element 1.

Note that, the third reflection layer R3 and the fourth reflection layer R4 may be integrated into one reflection layer. In this case, the integrated reflection layer reflects the light having the first wavelength and reflects the light having the second wavelength.

Fig. 13 is a diagram illustrating the laser element 1 obtained by providing the above-described optical element 9 in the laser element 1 in Fig. 12. The laser element 1 in Fig. 13 is an example in which the optical element 9 is arranged on the rear side of the optical axis with respect to the solid-state laser element 1. In the laser element 1 in Fig. 13, the convex mirror 10 of the optical element 9 is the fourth reflection layer R4. Therefore, the second resonator 12 causes the light having the second wavelength to resonate between the second reflection layer R2 in the solid-state laser element 1 and the fourth reflection layer R4, which is the convex mirror 10 of the optical element 9. The optical element 9 processes an end face of the transparent medium that transmits the light having the second wavelength into a concave shape to form the reflection film such as the dielectric multilayer film, thereby forming the convex mirror 10.

Note that, although the optical element 9 is arranged on the rear side of the optical axis with respect to the solid-state laser element 1 in Fig. 13, the second surface of the laser element 1 may be processed into a concave shape to form the fourth reflection layer R4 including the convex mirror 10.

In this manner, even in the laser element 1 not including the saturable absorber 4, by providing the optical element 9, the beam diameter of the light having the second wavelength can be increased, and the beam intensity in the solid-state laser medium 3 can be weakened, so that the optical damage can be suppressed.

### <<Application Example>>

The technology according to the present disclosure can be widely applied to a medical imaging system (hereinafter, also referred to as an electronic device), a ranging system such as a light detection and ranging (LiDAR) device, a light source for a laser processing device and the like. The medical imaging system is a medical system using an imaging technology, and is, for example, an endoscope system or a microscope system.

### [Endoscope system]

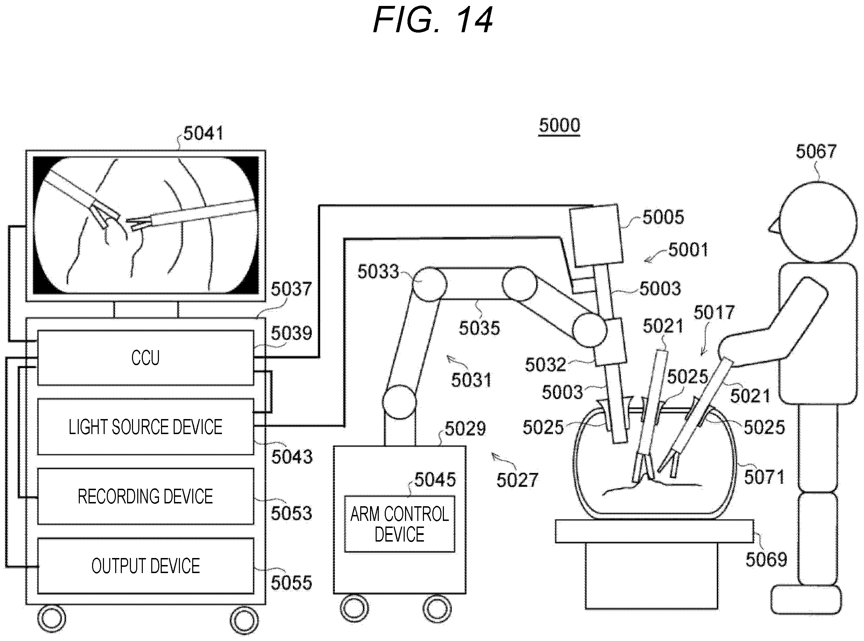

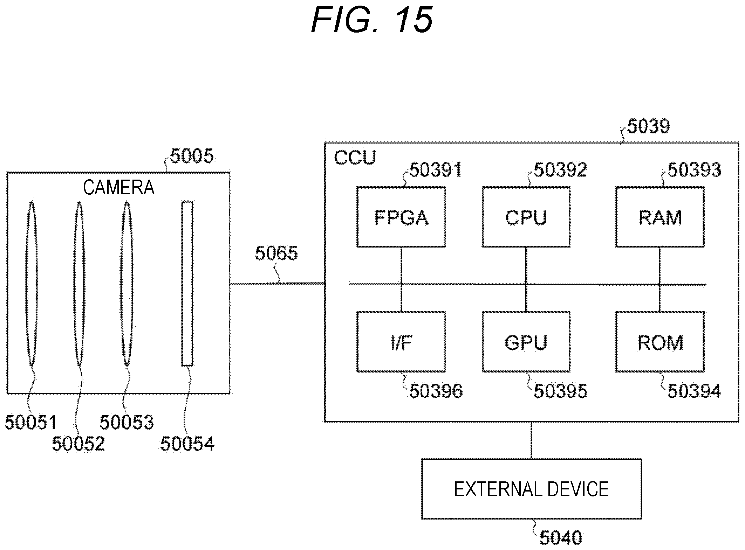

An example of an endoscope system is described with reference to Figs. 14 and 15. Fig. 14 is a diagram illustrating an example of a schematic configuration of an endoscope system 5000 to which the technology according to the present disclosure is applicable. Fig. 15 is a diagram illustrating an example of a configuration of an endoscope 5001 and a camera control unit (CCU) 5039. Fig. 14 illustrates a state in which an operator (for example, a surgeon) 5067 who is a surgery participant is performing surgery on a patient 5071 on a patient bed 5069 using the endoscope system 5000. As illustrated in Fig. 14, the endoscope system 5000 includes the endoscope 5001 that is a medical imaging device, the CCU 5039, a light source device 5043, a recording device 5053, an output device 5055, and a support device 5027 that supports the endoscope 5001.

In endoscopic surgery, insertion assisting tools called trocars 5025 are punctured into the patient 5071. Then, a scope 5003 connected to the endoscope 5001 and surgical tools 5021 are inserted into a body of the patient 5071 through the trocars 5025. The surgical tools 5021 include: an energy device such as an electric scalpel; and forceps, for example.

A surgical image that is a medical image in which the inside of the body of the patient 5071 is captured by the endoscope 5001 is displayed on a display device 5041. The operator 5067 performs a procedure on a surgical target using the surgical tools 5021 while viewing the surgical image displayed on the display device 5041. The medical image is not limited to the surgical image, and may be a diagnostic image captured during diagnosis.

### [Endoscope]

The endoscope 5001 is an imaging section for capturing the inside of the body of the patient 5071, and is, for example, as illustrated in Fig. 15, a camera 5005 including a condensing optical system 50051 for condensing incident light, a zooming optical system 50052 capable of optical zooming by changing a focal length of the imaging section, a focusing optical system 50053 capable of focus adjustment by changing the focal length of the imaging section, and a light receiving sensor 50054. The endoscope 5001 condenses the light through the connected scope 5003 on the light receiving sensor 50054 to generate a pixel signal, and outputs the pixel signal through a transmission system to the CCU 5039. The scope 5003 is an insertion part that includes an objective lens at a distal end and guides the light from the connected light source device 5043 into the body of the patient 5071. The scope 5003 is, for example, a rigid scope for a rigid endoscope and a flexible scope for a flexible endoscope. The scope 5003 may be a direct viewing scope or an oblique viewing scope. The pixel signal only needs to be a signal based on a signal output from a pixel, and is, for example, a raw signal or an image signal. The transmission system connecting the endoscope 5001 to the CCU 5039 may include a memory, and the memory may store parameters related to the endoscope 5001 and the CCU 5039. The memory may be disposed at a connection portion of the transmission system or on a cable. For example, the memory of the transmission system may store the parameters before shipment of the endoscope 5001 or the parameters changed when current is applied, and an operation of the endoscope may be changed based on the parameters read from the memory. A set of the camera and the transmission system may be referred to as an endoscope. The light receiving sensor 50054 is a sensor for converting the received light into the pixel signal, and is, for example, a complementary metal-oxide-semiconductor (CMOS) imaging sensor. The light receiving sensor 50054 is preferably an imaging sensor having a Bayer array capable of color imaging. The light receiving sensor 50054 is also preferably an imaging sensor having a number of pixels corresponding to a resolution of, for example, 4K (3840 horizontal pixels × 2160 vertical pixels), 8K (7680 horizontal pixels × 4320 vertical pixels), or square 4K (3840 or more horizontal pixels × 3840 or more vertical pixels). The light receiving sensor 50054 may be one sensor chip, or a plurality of sensor chips. For example, a prism may be provided to separate the incident light into predetermined wavelength bands, and the wavelength bands may be imaged by different light receiving sensors. A plurality of light receiving sensors may be provided for stereoscopic viewing. The light receiving sensor 50054 may be a sensor having a chip structure including an arithmetic processing circuit for image processing, or may be a sensor for time of flight (ToF). The transmission system is, for example, an optical fiber cable system or a wireless transmission system. The wireless transmission only needs to be capable of transmitting the pixel signal generated by the endoscope 5001, and, for example, the endoscope 5001 may be wirelessly connected to the CCU 5039, or the endoscope 5001 may be connected to the CCU 5039 via a base station in an operating room. At this time, the endoscope 5001 may transmit not only the pixel signal, but also simultaneously information (for example, a processing priority of the pixel signal and/or a synchronization signal) related to the pixel signal. In the endoscope, the scope may be integrated with the camera, and the light receiving sensor may be provided at the distal end of the scope.

### [Camera Control Unit (CCU)]

The CCU 5039 is a control device that integrally controls the connected endoscope 5001 and light source device 5043, and is, for example, an information processing device including an FPGA 50391, a CPU 50392, a RAM 50393, a ROM 50394, a GPU 50395, and an I/F 50396 as illustrated in Fig. 15. The CCU 5039 may control the display device 5041, the recording device 5053, and the output device 5055 connected to the CCU 5039 in an integrated manner. The CCU 5039 controls, for example, irradiation timing, irradiation intensity, and a type of an irradiation light source of the light source device 5043. The CCU 5039 also performs image processing, such as development processing (for example, demosaic processing) and correction processing, on the pixel signal output from the endoscope 5001, and outputs the processed image signal (for example, an image) to an external device such as the display device 5041. The CCU 5039 also transmits a control signal to the endoscope 5001 to control driving of the endoscope 5001. The control signal is information on an imaging condition such as a magnification or the focal length of the imaging section. The CCU 5039 may have a function to down-convert the image, and may be configured to be capable of simultaneously outputting a higher-resolution (for example, 4K) image to the display device 5041 and a lower-resolution (for example, high-definition (HD)) image to the recording device 5053.

The CCU 5039 may be connected to external equipment (such as a recording device, a display device, an output device, and a support device) via an IP converter for converting the signal into a predetermined communication protocol (such as the Internet Protocol (IP)). The connection between the IP converter and the external equipment may be established using a wired network, or a part or the whole of the network may be established using a wireless network. For example, the IP converter on the CCU 5039 side may have a wireless communication function, and may transmit the received image to an IP switcher or an output side IP converter via a wireless communication network, such as the fifth-generation mobile communication system (5G) or the sixth-generation mobile communication system (6G).

### [Light source device]

The light source device 5043 is a device capable of emitting the light having predetermined wavelength bands, and includes, for example, a plurality of light sources and a light source optical system for guiding the light of the light sources. The light sources are, for example, xenon lamps, light-emitting diode (LED) light sources, or laser diode (LD) light sources. The light source device 5043 includes, for example, the LED light sources corresponding to three respective primary colors of red (R), green (G), and blue (B), and controls output intensity and output timing of each of the light sources to emit white light. The light source device 5043 may include a light source capable of emitting special light used for special light observation, in addition to the light sources for emitting normal light for normal light observation. The special light is light having a predetermined wavelength band different from that of the normal light being light for the normal light observation, and is, for example, near-infrared light (light having a wavelength of 760 nm or longer), infrared light, blue light, or ultraviolet light. The normal light is, for example, the white light or green light. In narrow band imaging that is a kind of special light observation, blue light and green light are alternately emitted, and thus the narrow band imaging can image a predetermined tissue such as a blood vessel in a mucosal surface at high contrast using wavelength dependence of light absorption in the tissue of the body. In fluorescence observation that is a kind of special light observation, excitation light is emitted for exciting an agent injected into the tissue of the body, and fluorescence emitted by the tissue of the body or the agent as a label is received to obtain a fluorescent image, and thus the fluorescence observation can facilitate the operator to view, for example, the tissue of the body that is difficult to be viewed by the operator with the normal light. For example, in fluorescence observation using the infrared light, the infrared light having an excitation wavelength band is emitted to an agent, such as indocyanine green (ICG), injected into the tissue of the body, and the fluorescence light from the agent is received, whereby the fluorescence observation can facilitate viewing of a structure and an affected part of the tissue of the body. In the fluorescence observation, an agent (such as 5-aminolevulinic acid (5-ALA)) may be used that emits fluorescence in a red wavelength band by being excited by the special light in a blue wavelength band. The type of the irradiation light of the light source device 5043 is set by control of the CCU 5039. The CCU 5039 may have a mode of controlling the light source device 5043 and the endoscope 5001 to alternately perform the normal light observation and the special light observation. At this time, information based on a pixel signal obtained by the special light observation is preferably superimposed on a pixel signal obtained by the normal light observation. The special light observation may be an infrared light observation to observe a site inside the surface of an organ and a multi-spectrum observation utilizing hyperspectral spectroscopy. A photodynamic therapy may be incorporated.

### [Recording device]

The recording device 5053 is a device for recording the pixel signal (for example, an image) acquired from the CCU 5039, and is, for example, a recorder. The recording device 5053 records an image acquired from the CCU 5039 in a hard disk drive (HDD), a Super Density Disc (SDD), and/or an optical disc. The recording device 5053 may be connected to a network in a hospital to be accessible from equipment outside the operating room. The recording device 5053 may have a down-convert function or an up-convert function.

### [Display device]

The display device 5041 is a device capable of displaying the image, and is, for example, a display monitor. The display device 5041 displays a display image based on the pixel signal acquired from the CCU 5039. The display device 5041 may include a camera and a microphone to function as an input device that allows instruction input through gaze recognition, voice recognition, and gesture.

### [Output device]

The output device 5055 is a device for outputting the information acquired from the CCU 5039, and is, for example, a printer. The output device 5055 prints, for example, a print image based on the pixel signal acquired from the CCU 5039 on a sheet of paper.

### [Support device]

The support device 5027 is an articulated arm including a base 5029 including an arm control device 5045, an arm 5031 extending from the base 5029, and a holding part 5032 mounted at a distal end of the arm 5031. The arm control device 5045 includes a processor such as a CPU, and operates according to a predetermined computer program to control driving of the arm 5031. The support device 5027 uses the arm control device 5045 to control parameters including, for example, lengths of links 5035 constituting the arm 5031 and rotation angles and torque of joints 5033 so as to control, for example, the position and attitude of the endoscope 5001 held by the holding part 5032. This control can change the position or attitude of the endoscope 5001 to a desired position or attitude, makes it possible to insert the scope 5003 into the patient 5071, and can change the observed area in the body. The support device 5027 functions as an endoscope support arm for supporting the endoscope 5001 during the operation. Thus, the support device 5027 can play a role of a scopist who is an assistant holding the endoscope 5001. The support device 5027 may be a device for holding a microscope device 5301 to be described later, and can be called a medical support arm. The support device 5027 may be controlled using an autonomous control method by the arm control device 5045, or may be controlled using a control method in which the arm control device 5045 performs the control based on input of a user. The control method may be, for example, a master-slave method in which the support device 5027 serving as a slave device (replica device) that is a patient cart is controlled based on a movement of a master device (primary device) that is an operator console at a hand of the user. The support device 5027 may be remotely controllable from outside the operating room.

The example of the endoscope system 5000 to which the technology according to the present disclosure is applicable has been described above. For example, the technology according to the present disclosure may be applied to a microscope system.

### [Microscope system]

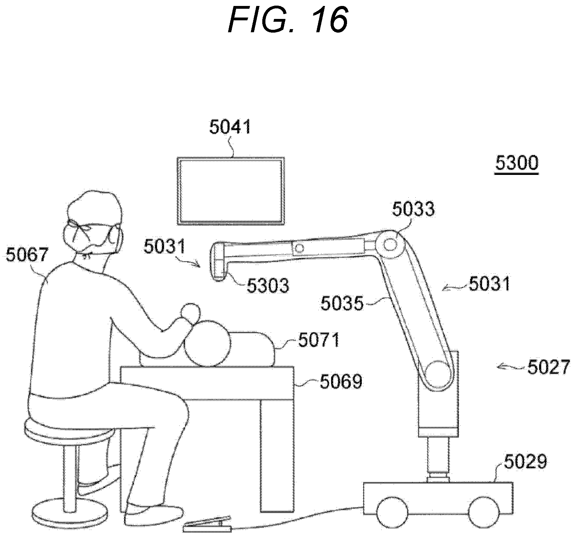

Fig. 16 is a diagram illustrating an example of a schematic configuration of a microscopic surgery system to which the technology according to the present disclosure is applicable. In the following description, the same components as those of the endoscope system 5000 will be denoted by the same reference numerals, and the description thereof will not be repeated.

Fig. 16 schematically illustrates a state in which the operator 5067 is performing surgery on the patient 5071 on the patient bed 5069 using a microscopic surgery system 5300. Note that, for the sake of simplicity, Fig. 16 does not illustrate a cart 5037 among the components of the microscopic surgery system 5300, and illustrates the microscope device 5301 instead of the endoscope 5001 in a simplified manner. The microscope device 5301 may refer to a microscope 5303 provided at the distal end of the links 5035, or may refer to the overall configuration including the microscope 5303 and the support device 5027.

As illustrated in Fig. 16, during the operation, the microscopic surgery system 5300 is used to display an image of a surgical site captured by the microscope device 5301 in a magnified manner on the display device 5041 installed in the operating room. The display device 5041 is installed in a position facing the operator 5067, and the operator 5067 performs various procedures, such as excision of an affected part, on the surgical site while observing the state of the surgical site using the image displayed on the display device 5041. The microscopic surgery system is used in, for example, ophthalmic operation and neurosurgical operation.

The respective examples of the endoscope system 5000 and the microscopic surgery system 5300 to which the technology according to the present disclosure is applicable have been described above. Systems to which the technology according to the present disclosure is applicable are not limited to such examples. For example, the support device 5027 can support, at the distal end thereof, another observation device or another surgical tool instead of the endoscope 5001 or the microscope 5303. Examples of the other applicable observation device include forceps, tweezers, a pneumoperitoneum tube for pneumoperitoneum, and an energy treatment tool for incising a tissue or sealing a blood vessel by cauterization. By using the support device to support the observation device or the surgical tool described above, the position thereof can be more stably fixed and the load of the medical staff can be lower than in a case where the medical staff manually supports the observation device or the surgical tool. The technology according to the present disclosure may be applied to a support device for supporting such a component other than the microscope.

The technology according to the present disclosure is suitably applicable to the surgical tools 5021 among the components described above. Specifically, by irradiating the affected site of the patient with a short-pulse laser pulse from the laser element 1 according to this embodiment, it is possible to more safely and reliably treat the affected site without damaging the periphery of the affected site.

Note that the present technology can also have the following configurations.
(1) A laser element including:
   a laminated semiconductor layer including a first reflection layer with respect to a first wavelength and an active layer that performs surface emission at the first wavelength;
   a laser medium arranged on a rear side of an optical axis of the laminated semiconductor layer and including a second reflection layer with respect to a second wavelength on a first surface facing the laminated semiconductor layer and a third reflection layer with respect to the first wavelength on a second surface on a side opposite to the first surface;
   a fourth reflection layer with respect to the second wavelength arranged on the second surface or arranged on a rear side of the optical axis with respect to the second surface;
   a first resonator that causes light having the first wavelength to resonate between the first reflection layer and the third reflection layer;
   a second resonator that causes light having the second wavelength to resonate between the second reflection layer and the fourth reflection layer; and
   an optical element arranged between the second reflection layer and the fourth reflection layer, the optical element that increases a beam diameter of the light having the second wavelength, in which
   the optical axis of the laminated semiconductor layer, an optical axis of the laser medium, and an optical axis of the optical element are coaxially arranged.
(2) The laser element according to (1), in which
   an end face on a side of the laser medium of the laminated semiconductor layer is joined to an end face on a side of the laminated semiconductor layer of the laser medium.
(3) The laser element according to (1) or (2), in which
   the optical element reflects or refracts at least a part of the light having the second wavelength so that the light having the second wavelength is not condensed.
(4) The laser element according to any one of (1) to (3), in which
   the optical element includes a dielectric multilayer film.
(5) The laser element according to any one of (1) to (4), in which
   the optical element includes a convex mirror that reflects at least a part of incident light, or a light refracting member that refracts at least a part of the incident light so that the incident light is not condensed.
(6) The laser element according to (5), in which
   in a case where the optical element includes the fourth reflection layer, the fourth reflection layer is the convex mirror, and in a case where the optical element is provided on a side closer to the second reflection layer than the fourth reflection layer, the optical element includes the light refracting member.
(7) The laser element according to (5), in which
   the optical element includes a transparent member that flattens a side of a surface opposite to a reflection surface of the convex mirror or a refraction surface of the light refracting member and transmits the light having the second wavelength.
(8) The laser element according to (7), further including:
   a light control member that is joined to the transparent member arranged on the side of the surface opposite to the refraction surface of the light refracting member and controls a refraction or polarization direction of the light having the second wavelength transmitted through the optical element.
(9) The laser element according to any one of (1) to (4), in which
   the optical element has a fine periodic structure that reflects or refracts at least a part of incident light in such a manner that the incident light is not condensed, and
   the fine periodic structure includes irregularities periodically arranged in a surface direction.
(10) The laser element according to (9), in which
   the fine periodic structure is a Fresnel lens, a meta lens, or a photonic crystal lens.
(11) The laser element according to any one of (1) to (4), in which
   the optical element includes a flat surface having a refractive index distribution in a surface direction that reflects or refracts at least a part of incident light in such a manner that the incident light is not condensed.
(12) The laser element according to any one of (1) to (11), further including:
   a saturable absorber including the fourth reflection layer on a third surface on a side opposite to the laser medium, in which
   the optical axis of the laminated semiconductor layer, the optical axis of the laser medium, the optical axis of the saturable absorber, and the optical axis of the optical element are coaxially arranged.
(13) The laser element according to (12), in which
   the laminated semiconductor layer, the laser medium, and the saturable absorber are integrally joined.
(14) The laser element according to (12), further including:
   a first transparent medium arranged between the laminated semiconductor layer and the laser medium, the first transparent medium that transmits the light having the first wavelength.
(15) The laser element according to (12), further including:
   a second transparent medium arranged between the laser medium and the saturable absorber, the second transparent medium that transmits the light having the second wavelength.
(16) The laser element according to any one of (12) to (15), in which
   the optical element is provided on at least one of the laser medium or the saturable absorber.
(17) The laser element according to (16), in which
   the optical element is provided on a surface or inside of the laser medium and the saturable absorber.
(18) The laser element according to (17), in which
   the optical element is provided along the third surface of the saturable absorber.
(19) The laser element according to any one of (12) to (15), in which
   the optical element is arranged between the laser medium and the saturable absorber.
(20) The laser element according to any one of (1) to (19), in which
   the fourth reflection layer is an output coupling mirror in the second resonator.
(21) The laser element according to any one of (1) to (20), in which
   the laminated semiconductor layer includes a fifth reflection layer with respect to the first wavelength, the fifth reflection layer arranged on a side closer to the laser medium than the first reflection layer, and
   the fifth reflection layer transmits a part of the light having the first wavelength.
(22) An electronic device including:
   a laser element; and
   a control unit that performs control to emit light from the laser element, in which
   the laser element includes:
      a laminated semiconductor layer including a first reflection layer with respect to a first wavelength and an active layer that performs surface emission at the first wavelength;
      a laser medium arranged on a rear side of an optical axis of the laminated semiconductor layer and including a second reflection layer with respect to a second wavelength on a first surface facing the laminated semiconductor layer and a third reflection layer with respect to the first wavelength on a second surface on a side opposite to the first surface;
      a fourth reflection layer with respect to the second wavelength arranged on the second surface or arranged on a rear side of the optical axis with respect to the second surface;
      a first resonator that causes light having the first wavelength to resonate between the first reflection layer and the third reflection layer;
      a second resonator that causes light having the second wavelength to resonate between the second reflection layer and the fourth reflection layer; and
      an optical element arranged between the second reflection layer and the fourth reflection layer, the optical element that increases a beam diameter of the light having the second wavelength, and
      the optical axis of the laminated semiconductor layer, an optical axis of the laser medium, and an optical axis of the optical element are coaxially arranged.

Aspects of the present disclosure are not limited to the above-described individual embodiments, but include various modifications that can be conceived by those skilled in the art, and the effects of the present disclosure are not limited to the above-described contents. That is, various additions, modifications, and partial deletions are possible without departing from the conceptual idea and spirit of the present disclosure derived from the matters defined in claims and equivalents thereof.

### REFERENCE SIGNS LIST

- 1: Laser element
- 2: Excitation light source
- 3: Solid-state laser medium
- 4: Saturable absorber
- 5: n-GaAs substrate
- 6: Cladding layer
- 7: Active layer
- 8: Cladding layer
- 9: Optical element
- 10: Convex mirror
- 11: First resonator
- 12: Q-switched solid-state laser resonator (second resonator)
- 13: Transparent medium
- 14: Flattening member
- 15: Photonic crystal layer
- 16: Fine periodic structure
- 17: GRIN lens
- 18: Laser array
- 21: Resist film
- 22: Photomask
- 23: Concave portion
- 24: Dielectric multilayer film
- 25: Alignment mark
- 26: Camera
- 27: First transparent medium
- 28: Second transparent medium
- 29: Transparent member

## Claims

1. A laser element comprising:
a laminated semiconductor layer including a first reflection layer with respect to a first wavelength and an active layer that performs surface emission at the first wavelength;
a laser medium arranged on a rear side of an optical axis of the laminated semiconductor layer and including a second reflection layer with respect to a second wavelength on a first surface facing the laminated semiconductor layer and a third reflection layer with respect to the first wavelength on a second surface on a side opposite to the first surface;
a fourth reflection layer with respect to the second wavelength arranged on the second surface or arranged on a rear side of the optical axis with respect to the second surface;
a first resonator that causes light having the first wavelength to resonate between the first reflection layer and the third reflection layer;
a second resonator that causes light having the second wavelength to resonate between the second reflection layer and the fourth reflection layer; and
an optical element arranged between the second reflection layer and the fourth reflection layer, the optical element that increases a beam diameter of the light having the second wavelength, wherein
the optical axis of the laminated semiconductor layer, an optical axis of the laser medium, and an optical axis of the optical element are coaxially arranged.

2. The laser element according to claim 1, wherein
an end face on a side of the laser medium of the laminated semiconductor layer is joined to an end face on a side of the laminated semiconductor layer of the laser medium.

3. The laser element according to claim 1, wherein
the optical element reflects or refracts at least a part of the light having the second wavelength so that the light having the second wavelength is not condensed.

4. The laser element according to claim 1, wherein
the optical element includes a dielectric multilayer film.

5. The laser element according to claim 1, wherein
the optical element includes a convex mirror that reflects at least a part of incident light, or a light refracting member that refracts at least a part of the incident light so that the incident light is not condensed.

6. The laser element according to claim 5, wherein
in a case where the optical element includes the fourth reflection layer, the fourth reflection layer is the convex mirror, and in a case where the optical element is provided on a side closer to the second reflection layer than the fourth reflection layer, the optical element includes the light refracting member.

7. The laser element according to claim 5, wherein
the optical element includes a transparent member that flattens a side of a surface opposite to a reflection surface of the convex mirror or a refraction surface of the light refracting member and transmits the light having the second wavelength.

8. The laser element according to claim 7, further comprising:
a light control member that is joined to the transparent member arranged on the side of the surface opposite to the refraction surface of the light refracting member and controls a refraction or polarization direction of the light having the second wavelength transmitted through the optical element.

9. The laser element according to claim 1, wherein
the optical element has a fine periodic structure that reflects or refracts at least a part of incident light in such a manner that the incident light is not condensed, and
the fine periodic structure includes irregularities periodically arranged in a surface direction.

10. The laser element according to claim 9, wherein
the fine periodic structure is a Fresnel lens, a meta lens, or a photonic crystal lens.

11. The laser element according to claim 1, wherein
the optical element includes a flat surface having a refractive index distribution in a surface direction that reflects or refracts at least a part of incident light in such a manner that the incident light is not condensed.

12. The laser element according to claim 1, further comprising:
a saturable absorber including the fourth reflection layer on a third surface on a side opposite to the laser medium, wherein
the optical axis of the laminated semiconductor layer, the optical axis of the laser medium, the optical axis of the saturable absorber, and the optical axis of the optical element are coaxially arranged.

13. The laser element according to claim 12, wherein
the laminated semiconductor layer, the laser medium, and the saturable absorber are integrally joined.

14. The laser element according to claim 12, further comprising:
a first transparent medium arranged between the laminated semiconductor layer and the laser medium, the first transparent medium that transmits the light having the first wavelength.

15. The laser element according to claim 12, further comprising:
a second transparent medium arranged between the laser medium and the saturable absorber, the second transparent medium that transmits the light having the second wavelength.

16. The laser element according to claim 12, wherein
the optical element is provided on at least one of the laser medium or the saturable absorber.

17. The laser element according to claim 16, wherein
the optical element is provided on a surface or inside of the laser medium and the saturable absorber.

18. The laser element according to claim 17, wherein
the optical element is provided along the third surface of the saturable absorber.

19. The laser element according to claim 12, wherein
the optical element is arranged between the laser medium and the saturable absorber.

20. The laser element according to claim 1, wherein
the fourth reflection layer is an output coupling mirror in the second resonator.

21. The laser element according to claim 1, wherein
the laminated semiconductor layer includes a fifth reflection layer with respect to the first wavelength, the fifth reflection layer arranged on a side closer to the laser medium than the first reflection layer, and
the fifth reflection layer transmits a part of the light having the first wavelength.

22. An electronic device comprising:
a laser element; and
a control unit that performs control to emit light from the laser element, wherein
the laser element includes:
a laminated semiconductor layer including a first reflection layer with respect to a first wavelength and an active layer that performs surface emission at the first wavelength;
a laser medium arranged on a rear side of an optical axis of the laminated semiconductor layer and including a second reflection layer with respect to a second wavelength on a first surface facing the laminated semiconductor layer and a third reflection layer with respect to the first wavelength on a second surface on a side opposite to the first surface;
a fourth reflection layer with respect to the second wavelength arranged on the second surface or arranged on a rear side of the optical axis with respect to the second surface;
a first resonator that causes light having the first wavelength to resonate between the first reflection layer and the third reflection layer;
a second resonator that causes light having the second wavelength to resonate between the second reflection layer and the fourth reflection layer; and
an optical element arranged between the second reflection layer and the fourth reflection layer, the optical element that increases a beam diameter of the light having the second wavelength, and
the optical axis of the laminated semiconductor layer, an optical axis of the laser medium, and an optical axis of the optical element are coaxially arranged.
